# EUROPEAN PATENT SPECIFICATION

(11) **EP 0 746 289 B1**
(45) Date of publication and mention of the grant of the patent: **07.04.1999**
(21) Application number: 92922366.7
(22) Date of filing: 21.10.1992
(51) Int. Cl.: A61F 6/04

(54) **CONDOM**
KONDOM
PRESERVATIF

(43) Date of publication of application: 11.12.1996
(73) Proprietor: SARIAN, Nabil Farid, Carlingford, NSW 2118 (AU)
(72) Inventor: SARIAN, Nabil Farid, Carlingford, NSW 2118 (AU)
(74) Representative: Bowman, Paul Alan
(86) International application number: AU9200559
(87) International publication number: WO9408535

(56) References cited:
- EP-A- 0 324 557
- AU-A- 4 289 789
- AU-A- 6 464 490
- DE-A- 1 566 365
- DE-C- 822 876
- FR-A- 1 561 079
- FR-A- 2 109 892
- GB-A- 1 473 261
- GB-A- 1 595 711
- US-A- 3 648 700
- US-A- 5 111 831

## Description

The present invention relates to birth control devices in the nature of condoms. In particular the invention relates to a condom having enhanced characteristics to prevent leakage of sperm.

Condoms are traditionally shaped so as to conform to the shape of the penis and fit snugly thereabout with the exception of a small void area provided in front of the urethra in order to accommodate the 2 - 5 millilitres of fluid usually produced upon ejaculation.

Such small void area or sac however does not always adequately contain the fluid produced and such fluid can work its way down the condom and leak from the base of the condom thereby permitting the sperm to enter the vagina.

Many of the sensitive nerve ending which give pleasure during intercourse are located down the shaft of the penis and of course a conventional condom shields these nerve endings from much the pleasurable sensations experienced during intercourse by both partners.

It is additionally thought that the transmission of diseases such as aids occur primarily due to ejaculated fluids. Indeed during withdrawal of the male penis from the vagina a vacuum is set up where by fluids are actually sucked into the male urethra.

Given the above mentioned primary method of transmission of diseases from male to female and vice versa it can be seen that it is unnecessary for the condom to extend down past the base of the head of the penis in order to prevent transmission of diseases provided of course that an adequate seal is present at the base of the head of the penis.

DE-1566365 disclose a condom comprising a sac to be positioned at the head of the penis defining the front of the condom, a cylindrical portion to accommodate the length of the penis and one elasticised band providing a seal for ejaculated semen.

EP-A-0324557 discloses a contraceptive prophylactic condom comprising a seminal receptacle having an open end and a closed end, a wider generally frustro-conical portion adjacent to and extending outwardly from the seminal receptacle corresponding approximately in size and shape to the tip of the male sex organ and having an indenture corresponding approximately to the indenture in the underside of the tip of the male sex organ, and adhesive means on an inner surface of the generally frustro-conical portion for sealing the condom to the tip of the male sex organ.

The present invention provides a condom comprising a sac to be positioned at the head of the penis defining the front of the condom and a first elasticised band providing a seal for ejaculated semen in which the condom further comprises a second elasticised band positioned towards the back of the condom relative to the first elasticised band and a first skirt positioned between the two elasticised bands, the first band being adapted to be positioned around the base of the head of the penis and the first skirt having been provided with an adhesive to bond to the skin of the penis.

The condom of the invention is preferably a "mini condom" adapted to cover only the head of the penis.

The condom of the invention provides more positive "seal" in order to prevent leakage of sperm. The mini condom reduces loss of sexual pleasure which turned many people of all ages from using condoms due to the fact the old full length condom designed to cover all the penis prevented sexual stimulation and pleasure to both male and female.

The invention will be described with reference to the accompanying drawings in which:
Figures 1A and 1B illustrate a condom of the invention, and
Figure 2 shows a condom of the invention in place on a penis.

The condom fabricated from a conventional rubberised material such as latex, is sized so as to fit around the head 5 of the penis with only a small skirt 9 extending beneath the relatively heavily elasticised area 3 on the condom such elasticised area 3 corresponding to the base of the head of the penis when the condom is in place upon an erect penis. The conventional sac 7 is still provided in order accommodate ejaculated fluid.

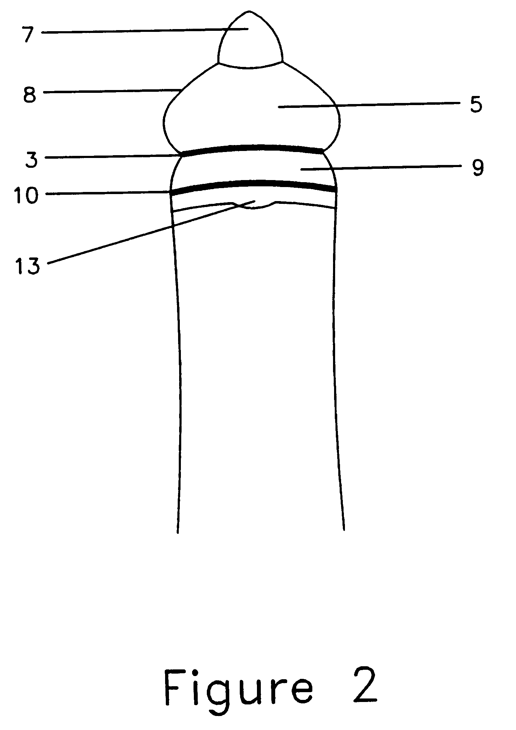

In order to make absolutely sure that the condom effects a positive seal to prevent leakage of ejaculated fluid a second elasticised annular seal 10 is provided adjacent the base 11 of the skirt. This elasticised seal being adapted to fit snugly about the relevant portion of the penis as depicted in FIG. 2. To yet further ensure the integrity of the seal of this condom the skirt area is provided with an adhesive so that the area between elasticised portions 3 and 10 is additionally bonded to the skin of the penis during the act of intercourse. A number of known adhesives may be appropriate including that of the type currently used in connection with other articles intended to adhere to human skin such as "Band Aids" TM.

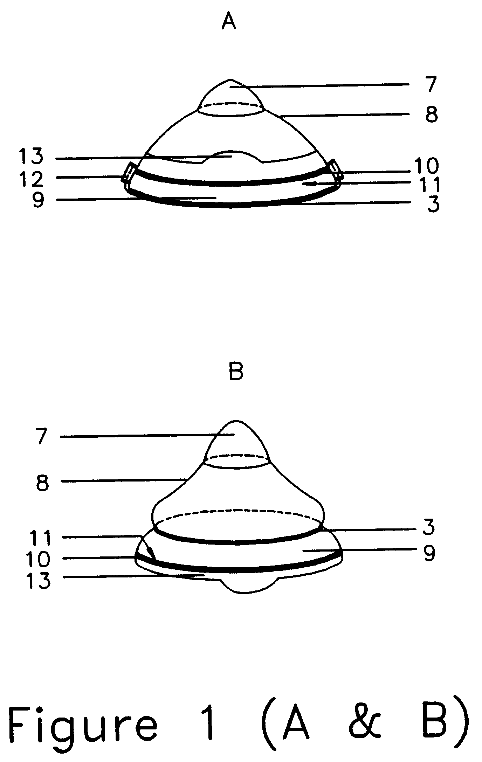

As will be observed from FIG. 1(A), the adhesive on the skirt may be protected prior to usage by the provision of tear off paper strips 12 as is in the case with "Band Aids" TM. The sac may contain a solidifying agent adapted to cause the ejaculated fluid to gel/or solidify thereby immobilising the sperm thereby preventing exit of such fluid from the sac or condom. Such solidifying agent lay additionally coat the other internal surfaces of the condom apart from sac 7. The sac and condom lay additionally of course contain spermicides of a conventional formulation.

The provision of a secondary skirt below the second elasticised portion 10 at the base of the skirt 9 may be provided in order to assist removal of the condom. This secondary skirt 13 would simply form a convenient portion of the condom which the user may grasp in order to peel the condom from the penis.

It will be appreciated that a "mini condom" in accordance with the present invention as described with reference to the embodiment depicted in Figures 1 and 2 hereof would enhance the pleasurable sensations experienced by the male during intercourse and may well therefore result in increased use of condoms.

The seals and solidifying agents described with reference to preferred embodiment of the present invention may also be used in conjunction with conventional condoms and it will be appreciated that the solidifying agent in particular would greatly reduce the incidence of bodily contact by all concerned parties with ejaculated fluid both during intercourse and after intercourse during the handling and disposal of the condom.

### PRECAUTIONS.

- "Mini Condom" best used to a penis free of ulcers, scratches or rashes.
- Adhesive material can cause allergy to certain individuals.
- In both conditions use our modified full length condom.
- Condom to be used only once, and to be disposed safely.

### HOW TO USE THE "MINI CONDOM" (very easy).

- Hold the "Mini Condom" with both thumb and index fingers from both angles, stretch a little place it on the erected penis till the base of the condom fits tightly underneath the rim of the penis head.
- Release one hand and use to press the sac to allow air out and then release the other hand.
- Peel protected paper from both sides of the skirt and pull down without touching adhesive part. To stick on the skin allow gentle pressure right around the skirt to secure seal.
- To remove simply hold the dry flaps front and back, lift up and peel.

## Claims

1. A condom comprising a sac (7) to be positioned at the head of the penis defining the front of the condom and a first elasticised band (3) providing a seal for ejaculated semen characterised in that the condom further comprises a second elasticised band (10) positioned towards the back of the condom relative to the first elasticised band (3) and a first skirt (9) positioned between the two elasticised bands (3, 10), the first band (3) being adapted to be positioned around the base of the head of the penis and the first skirt (9) having been provided with an adhesive to bond to the skin of the penis.

2. A condom as claimed in Claim 1 characterised in that it is adapted to cover only the head of the penis.

3. A condom as claimed in Claim 2 characterised in that the first skirt is essentially one centimetre.

4. A condom as claimed in Claims 2 or 3 characterised in that the condom further comprises a second skirt below the second elasticised band to assist the user to grasp and peel the condom from the penis.

5. A condom as claimed in Claim 4 characterised in that the second skirt is essentially one half centimetre.

6. A condom as claimed in any preceding Claim characterised in that the adhesive on the first skirt is protected prior to usage by a tear off paper strip.

7. A condom as claimed in any preceding Claim characterised in that the interior of the sac contains a solidifying agent to cause ejaculated fluid to gel or solidify thereby immobilising the sperm.

8. A condom as claimed in any preceding Claim characterised in that the interior of the sac contains a spermicide.

## Patentansprüche

1. Kondom, umfassend ein an der Eichel zu positionierendes Säckchen (7), welches das Vorderteil des Kondoms definiert, und ein erstes dehnbares Band (3), das eine Dichtung für ejakuliertes Sperma bereitstellt, dadurch gekennzeichnet, daß das Kondom des weiteren ein zweites dehnbares Band (10), das relativ zum ersten dehnbaren Band (3) hinten am Kondom angeordnet ist, und einen zwischen den zwei dehnbaren Bändern (3, 10) angeordneten ersten Kragen (9) umfaßt, wobei das erste Band (3) zum Positionieren um den Eichelansatz herum ausgeführt ist und der erste Kragen (9) mit einem Klebstoff versehen worden ist, um auf der Haut des Penis zu haften.

2. Kondom nach Anspruch 1, dadurch gekennzeichnet, daß es so ausgeführt ist, daß es nur die Eichel bedeckt.

3. Kondom nach Anspruch 2, dadurch gekennzeichnet, daß der erste Kragen im wesentlichen einen Zentimeter breit ist.

4. Kondom nach Anspruch 2 oder Anspruch 3, dadurch gekennzeichnet, daß das Kondom des weiteren einen zweiten Kragen unter dem zweiten dehnbaren Band aufweist, um es dem Benutzer zu erleichtern, das Kondom zu fassen und vom Penis abzuziehen.

5. Kondom nach Anspruch 4, dadurch gekennzeichnet, daß der zweite Kragen im wesentlichen einen halben Zentimeter breit ist.

6. Kondom nach einem der vorangehenden Ansprüche, dadurch gekennzeichnet, daß der Klebstoff auf dem ersten Kragen vor der Benutzung durch einen abziehbaren Papierstreifen geschützt wird.

7. Kondom nach einem der vorangehenden Ansprüche, dadurch gekennzeichnet, daß das Innere des Säckchens ein Verfestigungsmittel enthält, um zu bewirken, daß die ejakulierte Flüssigkeit geliert oder erstarrt, wodurch die Spermien unbeweglich gemacht werden.

8. Kondom nach einem der vorangehenden Ansprüche, dadurch gekennzeichnet, daß das Innere des Säckchens ein Spermizid enthält.

## Revendications

1. Condom comprenant un sac (7) devant être positionné à la tête du pénis et définissant l'avant du condom, et une première bande élastique (3) assurant l'étanchéité au sperme éjaculé, caractérisé en ce que le condom comprend en outre une deuxième bande élastique (10) positionnée vers l'arrière du condom par rapport à la première bande élastique (3) et une première jupe (9) positionnée entre les deux bandes élastiques (3, 10), la première bande (3) étant adaptée pour être positionnée autour de la base de la tête du pénis et la première jupe (9) ayant été munie d'un adhésif pour adhérer à la peau du pénis.

2. Condom selon la Revendication 1, caractérisé en ce qu'il est adapté pour ne couvrir que la tête du pénis.

3. Condom selon la Revendication 2, caractérisé en ce que la première jupe mesure essentiellement un centimètre.

4. Condom selon les Revendications 2 ou 3, caractérisé en ce que le condom comprend en outre une deuxième jupe en dessous de la deuxième bande élastique pour aider l'utilisateur à saisir le condom et à l'enlever du pénis.

5. Condom selon la Revendication 4, caractérisé en ce que la deuxième jupe mesure essentiellement un demi-centimètre.

6. Condom selon l'une quelconque des revendications précédentes, caractérisé en ce que l'adhésif de la première jupe est protégé avant usage par une bande de papier à arracher.

7. Condom selon l'une quelconque des Revendications précédentes, caractérisé en ce que l'intérieur du sac contient un agent solidifiant pour gélifier ou solidifier le liquide éjaculé et immobiliser ainsi le sperme.

8. Condom selon l'une quelconque des Revendications précédentes, caractérisé en ce que l'intérieur du sac contient un spermicide.
